Europäisches Patentamt

European Patent Office

Office européen des brevets

(19)

(11) Veröffentlichungsnummer: **0 070 393**
**B1**

(12) **EUROPÄISCHE PATENTSCHRIFT**

(45) Veröffentlichungstag der Patentschrift:
29.05.85

(21) Anmeldenummer: 82105350.1

(22) Anmeldetag: 18.06.82

(51) Int. Cl.⁴: **C 07 C 51/265,** C 07 C 63/04,
C 07 C 65/21, C 07 C 79/46,
C 07 C 51/47

(54) Verfahren zur Herstellung von aromatischen Monocarbonsäuren.

(30) Priorität: 16.07.81 DE 3128147

(43) Veröffentlichungstag der Anmeldung:
26.01.83 Patentblatt 83/4

(45) Bekanntmachung des Hinweises auf die Patenterteilung:
29.05.85 Patentblatt 85/22

(84) Benannte Vertragsstaaten:
**DE FR GB IT SE**

(56) Entgegenhaltungen:
**EP - A - 0 002 749**
**EP - A - 0 021 747**
**DE - A - 2 436 177**
**DE - A - 2 647 698**
**DE - A - 2 906 945**

(73) Patentinhaber: **DYNAMIT NOBEL
AKTIENGESELLSCHAFT, Postfach 1209,
D-5210 Troisdorf, Bez. Köln (DE)**

(72) Erfinder: **Feld, Marcel, Dr., Im Lochgarten 56,
D-5000 Köln 90 (DE)**

BUNDESDRUCKEREI BERLIN

## Beschreibung

Die Erfindung betrifft die Herstellung von aromatischen Monocarbonsäuren nach Verfahren der Oxidation von Toluol oder kernsubstituierten Toluolen der im Oberbegriff des Anspruchs 1 bezeichneten Art durch verbesserte Aufarbeitung.

Geeignete Ausgangsstoffe sind neben Toluol noch substituierte Toluole mit einem oder mehreren, gleichen oder verschiedenen, unter den Reaktionsbedingungen weitestgehend beständigen Substituenten wie beispielsweise Halogene, Aryl-, Nitro-, Alkoxy-, Aryloxy- und tert. Alkylgruppen.

Das Verfahren ist dadurch charakterisiert, daß Reaktions- und Aufarbeitungsbedingungen derart aufeinander abgestimmt sind, daß selbst im Falle eines Zielproduktes mit relativ hoher Löslichkeit in Essigsäure oder eines Ausgangsproduktes von vergleichsweise geringer Reinheit die aromatische Carbonsäure nach einem technisch sehr einfachen Verfahren in relativ hoher Ausbeute und hoher Reinheit gewonnen werden kann.

Es sind bereits zahlreiche Verfahren zur Oxidation arylständiger Methylgruppen mit Luftsauerstoff unter der wirkungsvollen Katalyse von Schwermetallsalzen, insbesondere Kobaltsalzen, von Bromiden als Kokatalysatoren und unter der Verwendung von Essigsäure als Lösungsmittel bekannt. Da die Reaktivität der Methylgruppe durch weitere Substituenten am Aromaten beeinflußt wird, ergeben sich für unterschiedlich substituierte Toluole auch unterschiedliche, optimale Oxidationsbedingungen. Dem wird bei einigen Verfahren zur Herstellung substituierter Benzoesäuren bereits durch die Auswahl geeigneter Parameter wie Temperatur, Druck, Katalysatorzusammensetzung und -konzentration Rechnung getragen. Während so den geeigneten Reaktionsbedingungen bei der Ausarbeitung der beschriebenen Oxidationsverfahren die notwendige Aufmerksamkeit zukommt, wird die für Ausbeute und Reinheit des Zielproduktes eines technischen Produktionsverfahrens mitentscheidende Art der möglichst einfachen, produktionsgemäßen Aufarbeitung der Reaktionsgemische insbesondere in solchen Fällen nicht hinreichend beachtet, wo die Herstellung in Essigsäure relativ gut löslicher Benzoesäuren beschrieben wird. Vor allem aber werden bei den bekannten Verfahren die Reaktionsbedingungen nicht den Forderungen einer unter wirtschaftlichen Gesichtspunkten günstigen, technisch leicht realisierbaren Aufarbeitung angepaßt.

Nach der EP-A1-0 002 749 werden mit 0,05 bis 2 Teilen von z. B. Essigsäure je Teil des Ausgangsstoffes in Gegenwart von Kobalt- und/oder Mangan-Katalysator aromatische Monocarbonsäuren hergestellt. Die Aufarbeitung nach der EP-A1-0 002 749 ist offenbar schwierig, denn diese erfolgt durch Kühlen, Trennen und Waschen sowie durch eine fraktionierte Destillation und Trennung von mehreren Fraktionen.

Nach der EP-A1-0 021 747 werden besonders Xylol zu Terephthalsäure in Gegenwart von Katalysatoren oxidiert, wobei weiteres Lösungsmittel wie Essigsäure, Wasser oder deren Mischungen nach der ersten Oxidationsstufe zur Verbesserung der Reinheit des Produktes zugegeben werden. Aromatische Monocarbonsäuren und die Art der Aufarbeitung sind nicht angesprochen.

Nach der DE-A1-2 647 698 wird zur Erhöhung der Ausbeute und Reinheit von Iso- oder Terephthalsäure bei Herstellung aus Xylolen wasserfreie Essigsäure der Aufarbeitung mit Gehalten des Katalysators zurückgeführt.

Nach der DE-A1-2 906 945 wird reine Terephthalsäure durch Gegenstromkontakt der heißen Suspension des Produktes in Essigsäure mit kalter wäßriger Essigsäure von höchstens 10°C in eine Kristallsuspension in praktisch reiner Essigsäure überführt, die die Abtrennung und Reinheit verbessert.

Monocarbonsäuren und der Zusammenhang von deren Herstellverfahren und der erfindungsgemäß verbesserten Aufarbeitung sind von diesem Stand der Technik nicht angesprochen.

Gegenstand der Erfindung ist ein Verfahren gemäß Patentanspruch 1. Die vorliegende Erfindung beschreibt ein technisch einfaches Gesamtverfahren zur Oxidation von Toluol und substituierten Toluolen und zur Isolierung der als Zielprodukte resultierenden Monocarbonsäuren. Es handelt sich bei diesem Verfahren um die Oxidation mit Sauerstoff bzw. einem sauerstoffhaltigen Gas, insbesondere Luft, bei einem erhöhten Druck von 5 bis 50 bar und einer Temperatur von 80 bis 180°C in Gegenwart von Essigsäure, eines löslichen Kobalt- und/oder Mangansalzes, die gegebenenfalls zusammen mit einem weiteren Schwermetallkatalysator eingesetzt werden können, und eines Bromids, wobei der Schwermetallkatalysator und der zu oxidierende Alkylaromat im molaren Verhältnis 0,003 : 1 bis 0,02 : 1 und das Bromid und der Schwermetallkatalysator im molaren Verhältnis 0,5 : 1 bis 2 : 1 eingesetzt werden.

Das erfindungsgemäße Verfahren ist durch eine besonders hohe Konzentration des Alkylaromaten bzw. durch eine besonders geringe Konzentration der Essigsäure im Ausgangsgemisch gekennzeichnet, in dem Alkylaromat und Essigsäure im Gewichtsverhältnis 0,5 : 1 bis 6 : 1, bei der Mehrzahl der Verfahren vorzugsweise im Gewichtsverhältnis 1 : 1 bis 3 : 1 zum Einsatz gebracht werden. Dabei ist das innerhalb der genannten Grenzen bevorzugte Gewichtsverhältnis von Alkylaromat und Essigsäure von der Löslichkeit des Zielproduktes und von der Art des Ausgangsproduktes abhängig, d. h., von der Neigung der betreffenden Ausgangs-, Zwischen- und Endprodukte zu Nebenreaktionen unter den jeweils erforderlichen Reaktionsbedingungen. Als wesentliches Kennzeichen des erfindungsgemäßen Verfahrens erfolgt erst nach beendeter Oxidation die zur Abtrennung des Zielproduktes aus dem Reaktionsgemisch erforderliche bzw. zur Erzielung einer hohen Ausbeute und Reinheit des Zielproduktes vorteilhafte Verdünnung mit einem geeigneten Lösungsmittel. Dabei kann zur Verdünnung des Reaktionsgemisches besonders vorteilhaft das Waschfiltrat eines vorausgegangenen, gleichartigen Oxidationsansatzes ganz oder teilweise verwendet werden.

Die vorteilhafte Verwendung der relativ geringen Essigsäurekonzentration während der Oxidation wird durch bekannte Verfahren nicht nahegelegt und steht sogar im Widerspruch zu der durch einige Patentschriften vermittelten Lehre. So wird in der Literatur mehrfach darauf hingewiesen, daß größere Wasserkonzentrationen bei der katalytischen Luftoxidation von Alkylaromaten stören. Nach DE-OS 1 768 899 sollte das Gewichtsverhältnis von Wasser zu Essigsäure nicht größer als 1 : 9 sein, nach DE-AS 1 418 852 kann sogar ein Wassergehalt von über 0,05 Gewichtsteilen pro Gewichtsteil Essigsäure bereits zum Oxidationsabbruch führen.

Eine Störung der Oxidation durch Wasser ist nun um so problematischer, als sich dieses als Oxidationsnebenprodukt mit fortschreitender Oxidation mehr und mehr anreichert. Um den störenden Einfluß des Wassers möglichst zu vermeiden, wird üblicherweise die bei der katalytischen Luftoxidation von Alkylaromaten als Lösungsmittel verwendete Essigsäure gegenüber dem zu oxidierenden Ausgangsprodukt in einer relativ hohen Konzentration eingesetzt. Dies entspricht der z. B. durch DE-OS 1 768 899, DE-AS 1 418 852 und JA 33 670/77 vermittelten Lehre. Zur Reduzierung der Wasserkonzentration kann aber auch, wie beispielsweise in JA 41 253/77 beschrieben, in Gegenwart von Acetanhydrid oxidiert oder aber auch ein Teil des Reaktionswassers analog DE-OS 1 768 899 und DE-OS 2 436 177 während der Oxidation destillativ entfernt werden.

Die übliche Verwendung relativ großer Lösungsmittelmengen in der Oxidationsstufe der Produktion aromatischer Monocarbonsäuren durch eine durch Schwermetalle und Bromide katalysierte Luftoxidation von Alkylaromaten in essigsaurer Lösung bringt nun in mehrfacher Hinsicht erhebliche Nachteile. So ist die während der Oxidation verwendete Lösungsmittelmenge mitbestimmend für die Dimensionierung des Reaktors, für den besonders korrosionsbeständige Materialien, vorzugsweise Titan, erforderlich sind. Für die diesbezüglich weniger beanspruchten Kristallisationsgefäße ist dagegen Edelstahl hinreichend beständig. Unter diesem Gesichtspunkt wäre bei gleicher Produktionskapazität ein kleiner dimensionierter Reaktor durchaus vorteilhaft.

Entscheidendere Nachteile bringt die Verwendung relativ großer Mengen Essigsäure aber bei der technischen Aufarbeitung der Reaktionsgemische. Viele aromatische Monocarbonsäuren, wie beispielsweise Benzoesäure, o-Chlorbenzoesäure, m-Nitrobenzoesäure, p-tert.Butylbenzoesäure oder Anissäure, sind in Essigsäure gut löslich. Dadurch wird die Isolierung der Zielprodukte in hoher Ausbeute erheblich erschwert. Die einfache Filtration der auf Raumtemperatur abgekühlten Reaktionsgemische würde zu beträchtlichen Ausbeuteverlusten führen. Um diese zu reduzieren, muß im einfachsten Fall das Reaktionsgemisch vor Isolierung des Zielproduktes eingeengt werden. Im Falle besonders hoher Löslichkeit des Zielproduktes wäre aber auch diese Maßnahme allein nicht ausreichend, da bereits bei der erforderlichen Wäsche des Filterkuchens mit Essigsäure große Produktverluste eintreten würden. Hier müßte dann ein zweites Lösungsmittel zum Auswaschen des Filterkuchens verwendet oder aber die Waschfiltrate zur Rückgewinnung der gelösten Produktanteile separat aufgearbeitet werden. In beiden Fällen würde die Aufarbeitung der Waschfiltrate einen erheblichen technischen Aufwand erfordern.

Auch bei der Herstellung von in Essigsäure weniger gut löslichen aromatischen Monocarbonsäure wird der technische Aufwand der Aufarbeitung von der Lösungsmittelmenge mitbestimmt, da die Filtrate zur Abtrennung des Reaktionswassers und Rückgewinnung der Essigsäure destillativ aufgearbeitet werden müssen.

Die Aufgabe, von der die Erfindung ausgeht, wird in dem Verfahren der eingangs bezeichneten Art durch die im Kennzeichen des Anspruchs 1 angegebenen Maßnahmen gelöst. Trotz des mehrfach bestätigten Störeinflusses größerer Wasserkonzentrationen wurde überraschend gefunden, daß bei der katalytischen Luftoxidation von Toluol oder kernsubstituierten Toluolen entsprechend dem erfindungsgemäßen Verfahren aromatische Monocarbonsäuren in hoher Ausbeute und hoher Reinheit erhalten werden können, wenn relativ geringe Mengen Essigsäure als Lösungs- bzw. Verdünnungsmittel eingesetzt werden. So gelang beispielsweise die Toluoloxidation noch bei einem molaren Toluol/Essigsäure-Verhältnis von > 1, wobei nach praktisch vollständigem Toluolumsatz das molare Verhältnis von Reaktionswasser zu Essigsäure den Wert 1 überstieg.

Die Vorteile des erfindungsgemäßen Verfahrens bezüglich der gewonnenen Ausbeute selbst im Falle relativ gut löslicher Zielprodukte und der technisch einfach realisierbaren Aufarbeitung der Reaktionsgemische sind dabei so groß, daß auch mögliche Nachteile der sehr geringen Lösungsmittelkonzentration bei der Oxidation in Form geringfügig verstärkter Nebenreaktionen bedeutungslos werden, sofern sie überhaupt eintreten. Entscheidend für ein technisches Verfahren ist ja nicht die Absolutmenge an gebildetem Zielprodukt, sondern die unter wirtschaftlichen Gesichtspunkten günstigste, d. h. nach einem möglichst einfachen Verfahren isolierbare Menge. In diesem Sinne sind beim erfindungsgemäßen Verfahren die Reaktionsbedingungen unter Verwendung einer relativ sehr geringen Lösungsmittelmenge den Forderungen nach einer technisch möglichst einfachen Aufarbeitung angepaßt.

Die Isolierung des Zielproduktes erfolgt beim erfindungsgemäßen Verfahren durch eine einfache Filtration nach einer der bekannten Filtrationsmethoden, beispielsweise unter Verwendung einer Filterzentrifuge. Selbst in den Fällen relativ gut löslicher Zielprodukte kann auf die technisch aufwendige vorherige Einengung des Reaktionsgemisches oder auf eine zweite Produktfiltration aus eingeengter Mutterlauge oder gar auf einen völligen Wechsel des Lösungsmittels verzichtet werden.

Beim erfindungsgemäßen Verfahren wird die Essigsäurekonzentration beim Reaktionsschritt so gering gewählt, daß zur Isolierung des Zielproduktes aus dem abgekühlten Reaktionsgemisch nach einer der üblichen Filtrationsmethoden die vorherige Verdünnung mit 0,2 bis 10, vorzugsweise 0,5 bis 5 Gewichtsteilen eines geeigneten Lösungsmittels oder Lösungsmittelgemisches pro Gewichtsteil bei der Reaktion verwendeter Essigsäure erforderlich ist. Die Bedingungen der Reaktionsgemischverdünnung sollten bezüglich Temperatur und Menge des Verdünnungsmittels so gewählt werden, daß dadurch keine Ausfällung zuvor gelösten Produktes erfolgt bzw. eventuell gefälltes Produkt durch anschließende Temperaturerhöhung wieder gelöst wird. Im Falle eines gut löslichen Zielproduktes sollte dieses nach erfolgter Verdünnung bei erhöhter Temperatur möglichst vollständig gelöst sein und erst im Verlauf der anschließenden Abkühlung auf Raumtemperatur bzw. auf eine Temperatur < Raumtemperatur aber oberhalb des Erstarrungspunktes der Mutterlauge auskristallisieren. Schwerlösliche Nebenprodukte können dann gegebenenfalls durch Filtration der heißen Reaktionslösung abgetrennt werden.

Auch wenn die Vorteile des erfindungsgemäßen Verfahrens besonders deutlich bei der Herstellung derjenigen aromatischen Monocarbonsäuren zum Ausdruck kommen, die in Essigsäure relativ gut löslich sind und daher auch im verdünnten, heißen Reaktionsgemisch gelöst vorliegen, bleibt das Verfahren jedoch nicht auf diese Fälle beschränkt, sondern kann auch erfolgreich und vorteilhaft zur Herstellung weniger gut löslicher Benzoesäuren angewendet werden, die im heißen, verdünnten Reaktionsgemisch bereits größtenteils ungelöst vorliegen.

Für die Verdünnung des Reaktionsgemisches nach beendeter Reaktion, die gemäß dem beschriebenen Verfahren zur Isolierung des Zielproduktes in hoher Reinheit aus einem bei Raumtemperatur noch fließfähigen Gemisch erforderlich ist, bestehen prinzipiell verschiedene Möglichkeiten. Dabei kann es als weiterer Vorteil des erfindungsgemäßen Verfahrens gewertet werden, daß für das Verdünnungsmittel keine Forderung nach Oxidationsbeständigkeit bzw. nach einem unter Oxidationsbedingungen nicht reaktionshemmenden Verhalten besteht. Voraussetzung für das Verdünnungsmittel wäre lediglich, daß es unter den Aufarbeitungsbedingungen nicht mit einem Produkt des Reaktionsgemisches reagiert, die Aufarbeitung nicht erschwert oder sich nicht auf Ausbeute und Reinheit des Zielproduktes nachteilig auswirkt.

Unter diesen Gesichtspunkten kämen als Verdünnungsmittel beispielsweise niedermolekulare aliphatische oder aromatische Kohlenwasserstoffe, halogenierte Kohlenwasserstoffe, Ester, Ketone, Nitrile und auch schwerer veresterbare Alkohole in Betracht. Durch die Möglichkeit einer nahezu freien Wahl des geeigneten Verdünnungsmittels kann das Verfahren den jeweils speziellen, durch die Art der Verunreinigungen und der Löslichkeit des Zielproduktes bestimmten Bedingungen bestens angepaßt werden.

Trotz dieser vorteilhaften breiten Auswahl des geeigneten Verdünnungsmittels wird bei der bevorzugten Form des erfindungsgemäßen Verfahrens im Hinblick auf die destillative Aufarbeitung der wäßrig-essigsauren Mutterlauge zur Verdünnung des Reaktionsgemisches eine wäßrig-verdünnte Essigsäure verwendet. Der entscheidende Vorteil dieses Verfahrens resultiert dann aus der Möglichkeit, die Verdünnung des Reaktionsgemisches mit dem an Zielprodukt praktisch gesättigten Waschfiltrat eines vorausgegangenen, gleichartigen Oxidationsansatzes vorzunehmen.

Die zweifache Verwendung eines erheblichen, vielfach sogar des größeren Teils der insgesamt erforderlichen Lösungsmittelmengen, zunächst zum Waschen des Zielproduktes und dann als Waschfiltrat zur Verdünnung des Reaktionsgemisches eines nachfolgenden Ansatzes, ist in mehrfacher Hinsicht vorteilhaft. In den Fällen gut löslicher Zielprodukte, wie beispielsweise Benzoesäure, m-Nitrobenzoesäure, o-Chlorbenzoesäure, p-tert.Butylbenzoesäure oder Anissäure, werden durch diese Maßnahme die lösungsbedingten Ausbeuteverluste stark reduziert. Dies gilt insbesondere dann, wenn wegen der Verwendung eines Ausgangsproduktes geringer Reinheit oder erheblicher Nebenreaktionen ein besonders gründliches Waschen des Zielproduktes erforderlich ist. Gerade bei den u. a. als Konservierungsmittel für Lebensmittel und zur Herstellung von Pharmaka und Farbstoffen verwendeten aromatischen Monocarbonsäuren wird eine hohe Reinheit gefordert.

Die Forderung nach einer hohen Reinheit der durch katalytische Luftoxidation von Alkylaromaten gewonnenen Carbonsäuren setzt im einfachsten Fall ein gründliches Auswaschen der aus möglichst großen Lösungsmittelmengen isolierten Zielprodukte zur Beseitigung von Katalysatorresten und Nebenprodukten voraus. Nebenprodukte sind dabei nicht nur im Falle eines Ausgangsproduktes geringer Reinheit zu beachten, sondern lassen sich bei der über mehrere Stufen radikalisch verlaufenden Reaktion prinzipiell nicht vollständig unterdrücken.

Die Isolierung des Zielproduktes aus einer großen Lösungsmittelmenge bei gleichzeitiger Verwendung größerer Waschflüssigkeitsmengen führt selbst im Falle einer in Essigsäure weniger gut löslichen substituierten Benzoesäure, wie beispielsweise der p-Nitro-, p-Chlor- oder p-Phenoxybenzoesäure noch zu deutlichen Ausbeuteverlusten. Im Falle besser löslicher Zielprodukte wird das Verfahren wegen einer zu geringen Ausbeute oder zu komplizierter Aufarbeitung unwirtschaftlich. Darüber hinaus belastet die Verwendung größerer Lösungsmittelmengen aber auch die destillative Aufarbeitung der Filtrate stark.

Ein zweiter wesentlicher Vorteil der zweifachen Verwendung eines erheblichen Teils der insgesamt erforderlichen Lösungsmittelmengen ist also durch die Reduzierung der destillativ aufzuarbeitenden Gesamtfiltrate gegeben. Für den bevorzugten Fall der Verwendung wäßrig-verdünnter Essigsäure als Waschflüssigkeit vereinfacht sich schließlich auch noch die destillative Filtrataufarbeitung beträchtlich, da nur ein relativ geringer Teil möglichst wasserfreier Essigsäure als Reaktionsmedium zurückgewonnen werden muß, während die Hauptmenge des Destillats als wäßrig-verdünnte Essigsäure abgetrennt und als Waschflüssigkeit wieder verwendet werden kann. Der Vorteil des Verfahrens hinsichtlich der Filtrataufarbeitung ist dabei unabhängig von der Löslichkeit des Zielproduktes und gilt somit auch für die Herstellung in Essigsäure relativ schwer löslicher aromatischer Monocarbonsäuren.

Die Vorteile des erfindungsgemäßen Verfahrens sollen an einigen Beispielen verdeutlicht werden. So kann nach diesem Verfahren entsprechend den Beispielen 1 und 2 selbst die in Essigsäure besonders gut lösliche Benzoesäure in hoher Reinheit und befriedigender Ausbeute nach einem technisch sehr einfachen Verfahren gewonnen werden. Durch Wiederverwendung des Waschfiltrats von Beispiel 1 zur Verdünnung des Reaktionsgemisches von Beispiel 2 wurde die Ausbeute von 82 auf 87% d. Th. gesteigert. Wird das Waschfiltrat des Beispiels 2 in gleicher Weise erneut eingesetzt, so wären bei diesem Versuch pro Gewichtsteil Benzoesäure nur ca. 2 Teile Filtrat destillativ aufzuarbeiten, von denen wiederum nur ca. 15% als möglichst wasserfreie Essigsäure für einen nachfolgenden Oxidationsansatz isoliert werden müssen.

Trotz relativ guter Löslichkeit von Benzoesäure in essigsauren Medien bietet damit das erfindungsgemäße Verfahren die Möglichkeit, Benzoesäure mit einem besonders geringen technischen Aufwand herzustellen. Ähnliche Vorteile des geringen technischen Aufwandes bei der Reaktion und Aufarbeitung durch Anpassung der Reaktions- an die Aufarbeitungsbedingungen bezüglich der Konzentrationsverhältnisse ergeben sich auch bei der Oxidation substituierter Toluole, sofern die Substituenten weder eine Oxidationsreaktion inhibieren noch selbst unter den Reaktionsbedingungen bevorzugt gegenüber der Methylgruppe oxidiert werden. Als in diesem Sinne geeignete Substituenten wären tertiäre Alkylgruppen, Halogene, Aryl-, Aryloxi-, Alkyloxi- und Nitrogruppen anzusehen.

Der Vorteil des erfindungsgemäßen Verfahrens wird besonders im Falle eines stark verunreinigten Ausgangsproduktes bei zugleich guter Löslichkeit des Zielproduktes deutlich, der am Beispiel der Oxidation eines nur 92%igen p-tert.-Butyltoluols demonstriert wird. Trotz der beträchtlichen Verunreinigung des Ausgangsproduktes durch das m-Isomere und andere, bei der Produktion von p-tert.Butyltoluol üblicherweise mit anfallende und destillativ nur schwer abtrennbare Verunreinigungen, die ein gründliches Waschen des Zielproduktes erfordern, kann die p-tert.Butylbenzoesäure nach dem erfindungsgemäßen Verfahren mit einer Reinheit von 99,9% bei einer Ausbeute von 95% d. Th. gewonnen werden.

Das Erreichen einer befriedigenden Ausbeute ist bei der katalytischen Luftoxidation von p-tert.Butyltoluol in essigsaurer Lösung durch die hohe Löslichkeit von p-tert.Butylbenzoesäure (24 g in 100 g Essigsäure bei 24° C) besonders erschwert. Zwar kann die Löslichkeit des Zielproduktes durch Verdünnung der essigsauren Lösung mit Wasser stark herabgesetzt werden, jedoch wird dabei die Ausbeute auf Kosten der Reinheit gesteigert, wobei braune, das Produkt verfärbende Nebenprodukte ein besonderes, nur schwer lösbares Problem darstellen, siehe das hierfür angegebene Vergleichs-Beispiel der Aufarbeitung des Reaktionsgemisches der Oxidation von p-tert.Butyltoluol mit Luftsauerstoff in Essigsäure.

Das erfindungsgemäße Verfahren bietet hier durch die geringe Lösungsmittelmenge während der Reaktion und die Verwendung von Waschfiltraten für die zur Produktisolierung erforderliche Verdünnung des Reaktionsgemisches eine technisch leicht realisierbare Lösung an, die, ausgehend von einem p-tert.Butyltoluol geringer Reinheit, die Darstellung von p-tert.Butylbenzoesäure in zugleich hoher Reinheit und hoher Ausbeute gestattet. Dabei sind pro Gewichtsteil Zielprodukt nur weniger als 1,8 Gewichtsteile Filtrate destillativ aufzuarbeiten, von denen wiederum nur weniger als 20% in Form möglichst wasserfreier Essigsäure für den Wiedereinsatz erhalten werden müssen. Verzichtet man auf die Verdünnung des Reaktionsgemisches nach beendeter Oxidation, so erstarrt das unter den Bedingungen der Beispiele 3 bis 6 resultierende Reaktionsgemisch beim Abkühlen auf Raumtemperatur zu einer unfiltrierbaren, festen Masse.

Noch deutlicher als im Falle der Herstellung von p-tert.Butylbenzoesäure aus einem p-tert.-Butyltoluol geringer Reinheit ist die für derartige Problemfälle besonders vorteilhafte Wirkung des erfindungsgemäßen Verfahrens bei der Oxidation von 2-Chlor-4-tert.butyltoluol. Das durch Kernchlorierung von technischem p-tert.Butyltoluol nach üblichen Methoden erhältliche 2-Chlor-4-tert.butyltoluol fällt als Gemisch mehrerer Isomerer an. Das in den Beispielen 9 bis 11 eingesetzte Produkt enthielt nach destillativer Reinigung nur 85,3% 2-Chlor-4-tert.butyltoluol neben 7,6% 3-Chlor-4-tert.butyltoluol, 5,2% 2-Chlor-5-tert.butyltoluol und 1,9% weitere Verunreinigungen. Die Gewinnung einer reinen 2-Chlor-4-tert.butylbenzoesäure wird, ausgehend von diesem stark verunreinigten Ausgangsprodukt, noch durch die besonders hohe Löslichkeit der aromatischen Carbonsäure in essigsauren Medien beträchtlich erschwert, von der sich in 100 g einer 75%igen Essigsäure bei 22°C noch 13,4 g lösen. Sogar unter derart ungünstigen Voraussetzungen konnte das Zielprodukt noch in einer Ausbeute von 85% d. Th. nach dem erfindungsgemäßen Verfahren gewonnen werden.

Die Erfindung wird durch die nachfolgenden Beispiele näher erläutert, ohne auf diese Beispiele beschränkt zu sein.

### Beispiel 1

Ein mit Rührer, Gaseinleitungsrohr, Temperaturfühler, Manometer und Druck-Rückflußkühler ausgestatteter, beheizbarer Autoklav aus Hastelloy C wurde mit 300 g Toluol, 100 g Essigsäure, 3 kg Kobaltacetat-Tetrahydrat [$Co(OAc)_2 \cdot 4 H_2O$] und 1,5 g NaBr beschickt. Durch diese Mischung wurde bei 125—130°C und einem Druck von 25 bar unter Rühren Luft mit einer konstanten Austrittsgeschwindigkeit von 3 l/Min. geleitet. Der Reaktionsverlauf wurde durch kontinuierliche Messung des Sauerstoffgehaltes im Abgas kontrolliert. Das Ende der Reaktion nach 220 Minuten gab sich dadurch zu erkennen, daß der $O_2$-Gehalt im Abgas wieder den Ausgangswert von 21% erreichte. Dann wurde die Lufteinleitung abgebrochen und nach Zugabe von 450 g 50%iger Essigsäure unter Rühren auf 23°C abgekühlt. Aus dem Reaktionsgemisch wurde das kristalline Zielprodukt unter Verwendung einer Drucknutsche abgetrennt, mit 450 g 50%iger Essigsäure gewaschen und getrocknet. Es wurden 325,5 g rein weiße Benzoesäure erhalten.

### Beispiel 2

Der im Beispiel 1 beschriebene Versuch wurde wiederholt, wobei zum Verdünnen des Reaktionsgemisches nach beendeter Reaktion an Stelle der reinen 50%igen Essigsäure das in Beispiel 1 beim Auswaschen des Zielproduktes erhaltene und von der Reaktions-Mutterlauge getrennt aufgefangene Waschfiltrat (504 g) verwendet wurde. Bei analoger Aufarbeitung wie im vorausgegangenen Versuch resultierten 346,5 g rein weißer Benzoesäure mit einer Reinheit von 99,7%.

### Beispiel 3

Analog Beispiel 1 wurden 300 g p-tert.Butyltoluol (93,3%ig) in Gegenwart vn 50 g Essigsäure, 3 g $Co(OAc)_2 \cdot 4 H_2O$ und 1,5 g NaBr bei 25 bar und 115—135°C unter Hindurchleiten von Luft mit einer Gasaustrittsgeschwindigkeit von 1,5 l/ Min. oxidiert. Die Sauerstoffaufnahme war nach einer Reaktionszeit von 300 Minuten beendet, worauf 450 g 74,5%iger Essigsäure zugesetzt, das verdünnte Reaktionsgemisch unter Rühren auf Raumtemperatur abgekühlt, filtriert und das Kristallisat mit 450 g 70%iger Essigsäure gewaschen wurde. Es resultierten 311,5 g p-tert.Butylbenzoesäure in Form weißer Kristalle mit einer gaschromatographisch bestimmten Reinheit von 99,6 Gew.-% bei einem Terephthalsäuregehalt von 0,16%.

### Beispiel 4

Beispiel 3 wurde unter Verdoppelung der Essigsäuremenge auf 100 g bei einer Reaktionstemperatur von 90 bis 110°C, einer Gasaustrittsgeschwindigkeit von 1,7 l/Min. und einer Reaktionszeit von 250 Minuten wiederholt. Das Reaktionsgemisch wurde mit 450 g 70%iger Essigsäure verdünnt und das Zielprodukt wie in Beispiel 3 beschrieben isoliert und gereinigt, wobei das Waschfiltrat (464,5 g) von der Mutterlauge (579,5 g) getrennt aufgefangen wurde. Der Versuch ergab 311 g p-tert.Butylbenzoesäure in Form rein weißer Kristalle mit einer Reinheit von >99,8% bei einem Terephthalsäuregehalt von <0,02%.

### Beispiel 5

Der zuvor beschriebene Versuch wurde wiederholt, wobei zur Verdünnung des Reaktionsgemisches nach beendeter Reaktion an Stelle der 450 g 70%iger Essigsäure das in Beispiel 4 erhaltene Waschfiltrat verwendet wurde. Die Ausbeute an rein weißer p-tert.Butylbenzoesäure betrug 320 g mit einer Reinheit von 99,9%.

### Beispiel 6

Analog den vorausgegangenen Versuchen wurden 400 g p-tert.Butyltoluol in Gegenwart von 200 g Essigsäure, 4 g $Co(OAc)_2 \cdot 4 H_2O$ und 2 g NaBr bei 100—150°C und einer Gasaustrittsgeschwindigkeit von 3 l/Min. innerhalb von 180 Minuten oxidiert. Das Reaktionsgemisch wurde mit 600 g 73%iger Essigsäure verdünnt und auf 25°C abgekühlt. Das Zielprodukt wurde in üblicher Weise isoliert und nacheinander mit je 600 g 73%iger Essigsäure und heißem Wasser (80°C) gewaschen. Dabei resultierten 404,5 g p-tert.Butylbenzoesäure als rein weiße Kristalle mit einer Reinheit von 99,9%.

### Vergleichs-Beispiel

Unter Einsatz von 300 g p-tert.Butyltoluol (93,3%ig), 400 g Essigsäure, 25 g $Co(OAc)_2 \cdot 4 H_2O$ und 2 g $NH_4Br$ wurde die Oxidation in der zuvor beschriebenen Weise bei 115—130°C und 3 l/Min. Gasaustritt durchgeführt. Nach beendeter Oxidation wurden 135 g Wasser zugesetzt. Das derart verdünnte Reaktionsgemisch wurde unter Rühren auf 25°C abgekühlt, filtriert und der Filterkuchen nacheinander zweimal mit je 100 g 70%iger Essigsäure und einmal mit 100 g Wasser gewaschen und dann getrocknet. Es wurden 299 g bräunlich gefärbtes Produkt mit einer Reinheit von 99,2% erhalten.

### Beispiel 7

Analog Beispiel 1 wurden 300 g o-Chlortoluol in Gegenwart von 100 g Essigsäure, 3 g Co(OAc)$_2$ · 4 H$_2$O und 1,5 g NaBr bei einem Druck von 25 bar, einer Temperatur von 110—130°C und einer Gasaustrittsgeschwindigkeit von 3 l/Min. oxidiert. Nach beendeter Oxidation wurden 450 g 50%ige Essigsäure zugesetzt, auf 25°C abgekühlt, filtriert und der Filterkuchen mit 750 g 50%iger Essigsäure gewaschen. Dabei wurden Mutterlauge (314,5 g) und Waschfiltrate (693,5 g) getrennt aufgefangen. Der getrocknete Filterkuchen ergab 318,5 g o-Chlorbenzoesäure.

### Beispiel 8

Der in Beispiel 7 beschriebene Versuch wurde wiederholt, wobei das Reaktionsgemisch nach beendeter Reaktion allerdings mit 660 g des Waschfiltrats des vorausgegangenen Versuches verdünnt und der Filterkuchen nur mit 600 g 50%iger Essigsäure gewaschen wurde. Erhalten wurden 335,5 g o-Chlorbenzoesäure.

### Beispiel 9

Analog Beispiel 1 wurde 2-Chlor-4-tert.butyltoluol oxidiert, das durch Kernchlorierung eines technischen p-tert.Butyltoluols unter Verwendung von FeCl$_3$ als Katalysator erhalten worden war. Das Chlorierungsprodukt enthielt nach destillativer Reinigung nur 85,3% 2-Chlor-4-tert.butyltoluol neben 7,6% 3-Chlor-4-tert.butyltoluol, 5,2% 2-Chlor-5-tert.butyltoluol und 1,9% weitere Verunreinigungen. 350 g dieses Produktgemisches wurden nach Zusatz von 100 g Essigsäure, 3 g Co(OAc)$_2$ · 4 H$_2$O und 1,5 g NaBr bei 25 bar unter Hindurchleiten von Luft mit einer Austrittsgeschwindigkeit von 3 l/Min. innerhalb von 130 Minuten bei 130°C oxidiert. Nach beendeter Reaktion wurde mit 275 g Essigsäure und 225 g Wasser verdünnt, unter Rühren auf 3°C abgekühlt, filtriert, der Filterkuchen mit 500 g einer 55%igen Essigsäure bei 3°C gewaschen und im Luftstrom getrocknet. Es wurden 349 g gelbes Reaktionsprodukt mit einem Schmelzbereich von 98—108°C und einer gaschromatographisch bestimmten Reinheit von 96,9% erhalten.

### Beispiel 10

Der in Beispiel 9 beschriebene Versuch wurde bei geringfügig reduzierter Reaktionstemperatur von 125°C unter geänderten Aufarbeitungsbedingungen wiederholt. Nach beendeter Oxidation wurden hier nur 225 g Essigsäure und 75 g Wasser zugesetzt. Nach der Filtration des auf 3°C abgekühlten, verdünnten Reaktionsgemisches wurde mit 300 g 75%iger Essigsäure bei 3°C gewaschen, wobei Mutterlauge (419,5 g) und Waschfiltrat (360 g) getrennt aufgefangen wurden. Es resultierten 271 g 2-Chlor-4-tert.butylbenzoesäure als weißes Kristallisat mit einem Schmelzpunkt von 117°C und einer gaschromatographisch bestimmten Reinheit von 99,5%.

### Beispiel 11

Beispiel 10 wurde unter Verwendung von 355 g des bei diesem Versuch erhaltenen Waschfiltrats zur Verdünnung des Reaktionsgemisches nach beendeter Reaktion wiederholt. Es wurden 296 g 2-Chlor-4-tert.butylbenzoesäure der gleichen Qualität wie in Beispiel 10 erhalten.

### Beispiel 12

Entsprechend Beispiel 1 wurden 200 g p-Phenoxitoluol in Gegenwart von 200 g Essigsäure, 3 g Co(OAc)$_2$ · 4 H$_2$O und 1,5 g NaBr bei einem Druck von 25 bar, einem Gasaustritt von 3 l/Min. und einer Temperatur von 110—115°C innerhalb von 95 Minuten oxidiert. Nach beendeter Reaktion erstarrte das Oxidationsgemisch beim Abkühlen auf Raumtemperatur zu einer festen Masse. Es wurde mit 400 g 90%iger Essigsäure verdünnt, auf Rückflußtemperatur gebracht und die dabei resultierende klare Lösung unter Rühren auf Raumtemperatur abgekühlt. Die nunmehr erhaltene rühr- und fließfähige Suspension wurde filtriert und der Filterkuchen mit 400 g 90%iger Essigsäure gewaschen, wobei Mutterlauge (530 g) und Waschfiltrat (433,5 g) getrennt aufgefangen wurden. Der getrocknete Filterkuchen ergab 204 g p-Phenoxibenzoesäure mit einer Reinheit von 99,9%.

### Beispiel 13

Der in Beispiel 12 beschriebene Versuch wurde wiederholt, wobei zur Verdünnung des Reaktionsgemisches nach beendeter Oxidation an Stelle der 400 g 90%iger Essigsäure die 433,5 g Waschfiltrat des Beispiels 12 verwendet wurden. Der Versuch ergab eine Ausbeute von 209,5 g p-Phenoxibenzoesäure mit einer Reinheit von 99,9%.

## Beispiel 14

Beispiel 12 wurde unter Einsatz von 400 g p-Phenoxitoluol, 300 g Essigsäure, 5 g Co(OAc)$_2$ · 4 H$_2$O und 2,5 g NaBr bei einer Reaktionstemperatur von 118—125°C und einer Reaktionszeit von 150 Minuten wiederholt, wobei zur Verdünnung des Reaktionsgemisches nach beendeter Oxidation und zum Auswaschen des Zielproduktes nach der Druckfiltration jeweils 700 g 80%iger Essigsäure verwendet wurden. Als Ergebnis resultierten 419,5 g p-Phenoxibenzoesäure gleicher Reinheit wie in den beiden zuvor beschriebenen Versuchen.

## Beispiel 15

Beispiel 14 wurde unter Verwendung der dabei erhaltenen 693,5 g Waschfiltrat zur Verdünnung des Reaktionsgemisches nach beendeter Reaktion wiederholt, wobei 425,5 g p-Phenoxibenzoesäure mit einer Reinheit von 99,9% erhalten wurden.

## Beispiel 16

Entsprechend Beispiel 1 wurden 250 g m-Nitrotoluol in Gegenwart von 150 g Essigsäure, 4,5 g Co(OAc)$_2$ · 4 H$_2$O und 2 g NaBr bei einem Druck von 25 bar, einer Temperatur von 135—145°C und einer Gasaustrittsgeschwindigkeit von 3 l/Min. innerhalb von 200 Minuten oxidiert. Das mit 400 g 69%iger Essigsäure verdünnte Reaktionsgemisch wurde unter Rühren auf 5°C abgekühlt, filtriert, 2mal mit 125 g 50%iger Essigsäure bei 5°C und einmal mit 150 g Wasser gewaschen, wobei die Mutterlauge (514,5 g) und die vereinigten Waschfiltrate (441 g) getrennt aufgefangen wurden. Nach dem Trocknen resultierten 230 g m-Nitrobenzoesäure.

## Beispiel 17

Der als Beispiel 16 beschriebene Versuch wurde wiederholt, wobei zur Verdünnung des Reaktionsgemisches nach beendeter Oxidation die 441 g Waschfiltrat des Vorversuches verwendet wurden. Es wurden 245 g m-Nitrobenzoesäure erhalten.

## Patentansprüche

1. Verfahren zur Herstellung von aromatischen Monocarbonsäuren durch Oxidation von Toluol oder kernsubstituierten Toluolen mit unter Reaktionsbedingungen inerten Substituenten in Gegenwart von Essigsäure als Verdünnungsmittel bei einem Gewichtsverhältnis von 0,5 : 1 bis 6 : 1, von zu oxidierendem Alkylaromat und Essigsäure mit Sauerstoff bzw. einem Sauerstoff enthaltenden Gas in flüssiger Phase bei einer Temperatur von 80 bis 180°C und einem Druck von 5 bis 50 bar und einem löslichen Kobalt- und/oder Mangansalz in Kombination mit einem Bromid als Katalysator, bei einem molaren Verhältnis des Schwermetallkatalysators zu dem Alkylaromaten von 0,003 : 1 bis 0,02 : 1 und einem molaren Verhältnis des Bromids und des Schwermetalls von 0,5 : 1 bis 2 : 1, dadurch gekennzeichnet, daß bei der Aufarbeitung des Reaktionsgemisches pro Gewichtsteil der bei der Oxidation verwendeten Essigsäure die zur Isolierung des Zielproduktes durch Fest-Flüssig-Trennung des abgekühlten Reaktionsgemisches erforderliche Verdünnung mit 0,2 bis 10, vorzugsweise 0,5 bis 5 Gewichtsteilen einer wäßrig-verdünnten Essigsäure erfolgt.

2. Verfahren nach Anspruch 1, dadurch gekennzeichnet, daß zur Verdünnung des Reaktionsgemisches das Waschfiltrat eines vorausgegangenen Oxidationsansatzes mit den gleichen Ausgangsprodukten verwendet wird.

3. Verfahren nach einem der Ansprüche 1 bis 2, dadurch gekennzeichnet, daß die Verdünnung des Reaktionsgemisches nach beendeter Reaktion bei erhöhter Temperatur von 80 bis 180°C vorgenommen bzw. das Reaktionsgemisch nach erfolgter Verdünnung auf 80 bis 180°C erhitzt wird.

4. Verfahren nach einem der Ansprüche 1 bis 3, dadurch gekennzeichnet, daß p-tert.Butylbenzoesäure aus p-tert.Butyltoluol hergestellt wird.

5. Verfahren nach einem der Ansprüche 1 bis 3, dadurch gekennzeichnet, daß p-Phenoxibenzoesäure aus p-Phenoxitoluol hergestellt wird.

## Claims

1. Process for the preparation of aromatic monocarboxylic acids by oxidation of toluene or nuclearly substituted toluenes with substituents in inert reaction conditions in the presence of acetic acid as diluent in a weight ratio of 0.5 : 1 to 6 : 1 of alkyl aromatic to be oxidised and acetic acid, using oxygen or an oxygen-containing gas in the liquid phase at a temperature of 80 to 180°C and a pressure of 5 to 50 bar and a soluble cobalt and/or manganese salt in combination with a bromide as catalyst, in a molar ratio of heavy metal alkyl catalyst to the alkyl aromatics of 0.003 : 1 to 0.02 : 1 and a molar ratio of the bromide and the heavy metal of 0.5 : 1 to 2 : 1, characterised in that, on working up of the reaction mixture, the required dilution of the cooled reaction mixture for isolation of the target product by solid liquid separation taktes place with 0.2 to 10, preferably 0.5 to 5, parts by weight of an aqueous-diluted acetic acid per part by weight of the acetic acid used in the oxidation.

2. Process according to claim 1, characterised in that, for dilution of the reaction mixture, the washing filtrate of a previous oxidation batch with the same starting materials is used.

3. Process according to one of claims 1 or 2, characterised in that the dilution of the reaction mixture after the end of the reaction takes place at elevated temperature of 80 to 180°C or the reaction mixture is heated to 80 to 180°C after dilution takes place.

4. Process according to one of claims 1 to 3, characterised in that p-tert. butylbenzoic acid is produced from p-tert. butyltoluene.

5. Process according to one of claims 1 to 5, characterised in that p-phenoxybenzoic acid is produced from p-phenoxytoluene.

**Revendications**

1. Procédé de préparation d'acides monocarboxyliques aromatiques par oxydation du toluène ou de toluènes substitués sur le noyau par des substituants inertes dans les conditions de réaction, en présence d'acide acétique comme diluant, selon un rapport pondéral de 0,5 : 1 à 6 : 1 entre l'alkyl-aromatique à oxyder et l'acide acétique, par de l'oxygène ou un gaz contenant de l'oxygène, en phase liquide à une température de 80 à 180°C et sous une pression de 5 à 50 bars et avec, comme catalyseur, un sel soluble de cobalt et/ou de manganèse en combinaison avec un bromure, selon un rapport molaire du catalyseur de métal lourd à l'alkyl-aromatique de 0,003 : 1 à 0,02 : 1 et selon un rapport molaire du bromure et du métal lourd compris entre 0,5 : 1 et 2 : 1, procédé caractérisé en ce que, lors du traitement du mélange réactionnel, on effectue la dilution nécessaire pour l'isolement du produit, que l'on veut obtenir par séparation solide/liquide du mélange réactionnel refroidi, en utilisant 0,2 à 10, avantageusement 0,5 à 5, parties en poids d'un acide acétique aqueux dilué, par partie en poids de l'acide acétique utilisé lors de l'oxydation.

2. Procédé selon la revendication 1, caractérisé en ce que, pour diluer le mélange réactionnel, on utilise le filtrat de lavage d'une charge précédente d'oxydation comportant les mêmes produits de départ.

3. Procédé selon l'une des revendications 1 et 2, caractérisé en ce qu'on effectue la dilution du mélange réactionnel, après achèvement de la réaction, à une température accrue se situant entre 80 et 180°C ou en ce qu'on chauffe le mélange réactionnel, après réalisation de la dilution, jusqu'à 80 à 180°C.

4. Procédé selon l'une des revendications 1 à 3, caractérisé en ce qu'on prépare l'acide p-tertiobutyl benzoïque à partir du p-tertiobutyl toluène.

5. Procédé selon l'une des revendications 1 à 3, caractérisé en ce qu'on prépare l'acide p-phénoxy benzoïque à partir du p-phénoxy toluène.